# EUROPEAN PATENT SPECIFICATION

(11) **EP 3 329 846 B1**
(45) Date of publication and mention of the grant of the patent: **14.07.2021**
(21) Application number: 16830478.0
(22) Date of filing: 25.07.2016
(51) Int. Cl.: A61B 5/1172, C08F 2/44, C08F 2/48, C08F 122/32, C08K 5/5419, C09D 4/00

(54) **COMPOSITION FOR DETECTING LATENT FINGERPRINT**
ZUSAMMENSETZUNG ZUM ERFASSEN EINES LATENTEN FINGERABDRUCKS
COMPOSITION PERMETTANT DE DÉTECTER UNE EMPREINTE DIGITALE LATENTE

(30) Priority: 29.07.2015 JP 2015150075
(43) Date of publication of application: 06.06.2018
(73) Proprietor: Toagosei Co., Ltd., Minato-ku Tokyo 105-8419 (JP)
(72) Inventor: SHIMODA Osamu, Kobe-shi Hyogo 650-0011 (JP); TAKAGI, Yoshiyuki, Kanazawa-shi Ishikawa 9208203 (JP); MATSUMURA Motohiro, Kobe-shi Hyogo 650-0011 (JP); ANDO Yushi, Tokyo 105-8419 (JP)
(74) Representative: TBK
(86) International application number: PCT/JP2016/071699
(87) International publication number: WO 2017/018373

(56) References cited:
- WO-A2-2007/022377
- JP-A- H0 415 044
- JP-A- H06 165 771
- JP-A- S61 288 836
- JP-A- S63 108 010
- JP-A- S63 275 609
- JP-A- 2010 213 837
- US-A- 4 837 260
- US-A- 5 902 443
- US-A1- 2010 047 433

## Description

### [TECHNICAL FIELD]

The present invention relates to detecting technique of a latent fingerprint in the field of identification.

### [BACKGROUND ART]

A latent fingerprint is a fingerprint impressed in the form of a raised portion of the skin (skin small ridge; hereinafter, also referred to as a "fingerprint ridge") at a time when a substance secreted from a fingertip sticks to an article (hereinafter, also referred to as a "specimen") left at a crime scene. The substance secreted from a fingertip includes an inorganic component such as sodium chloride, potassium chloride and calcium chloride, lactic acid, an amino acid, uric acid, lipids, proteins, vitamins and so on, in addition to water as a main component. Since the latent fingerprint cannot be identified as it is, a chemical reaction or physical adsorption reaction between a fingertip secretory component and a detection substance has been used in identification. Specific examples of the reaction include a solid method in which a fine powder composed of aluminum, iron oxide, muscovite, kaolin or the like is subjected to physically adsorbing with water or a lipid which is a component of a fingerprint ridge to visualize; a method in which a solution containing ninhydrin, silver nitrate, DFO (1, 8-diazafluorein-9-one) or the like is used to react a lipid with an amino acid, a salt or the like to develop a color; a liquid method in which 2-cyanoacrylate is adsorbed to and reacted with water or a lipid; and a gas method in which an iodine gas, a 2-cyanoacrylate gas or the like is reacted with a lipid.

In a case where a latent fingerprint is detected by a liquid method using 2-cyanoacrylate among the above-described methods, a solution containing 2-cyanoacrylate is used. For example, Patent Document 1 discloses a liquid composition including 2-cyanoacrylate, rhodamine 6G, trichloroethane and chloroform, and suggests that an organic solvent such as a hydrocarbon, other halogenated hydrocarbon, an ether, an ester, an alcohol and a ketone may be used in place of trichloroethane or chloroform; and Patent Document 2 discloses a fingerprint detecting liquid composition comprising a cyanoacrylate and an aliphatic hydrocarbon solvent.

### [PRIOR TECHNICAL DOCUMENT]

### [PATENT DOCUMENT]

[Patent Document 1] JP-A Sho 63-161939
[Patent Document 2] JP-H-06-165771

### [SUMMARY OF THE INVENTION]

### [PROBLEMS THAT THE INVENTION IS TO SOLVE]

A solution containing 2-cyanoacrylate is suitable for detection of a latent fingerprint. However, a stuck latent fingerprint depends on a surface shape of a constituent member (hereinafter, also referred to as a "fingerprint support member") of a specimen, and therefore when a surface of the fingerprint support member has a portion with a fine projection (protrusion) and recess in sequence, a fingertip secretory component remains only at the tip of the projection. When the composition in the Patent Document 1 is used for such a specimen, the fingertip secretory component flows from the projection to recess, so that the latent fingerprint may be deformed or eliminated, leading to insufficient detection of the latent fingertip. In addition, some of organic solvents contained in the composition of the Patent Document 1 cause dissolution, swelling or degeneration of a constituent material of the fingerprint support member, and the specimen may be limited.

An object of the present invention is to provide a composition which ensures that not only when a latent fingerprint including a fingertip secretory component is formed on a surface of a flat fingerprint support member but also when a latent fingerprint is formed only on a surface of a projection on a fingerprint support member having a projection or recess on the outermost surface, deformation or elimination of the latent fingerprint is suppressed to retain a latent fingerprint pattern, so that the latent fingerprint can be efficiently detected by photoirradiation.

### [MEANS FOR SOLVING THE PROBLEMS]

The present inventors have extensively conducted studies on a liquid composition which suppresses deformation or elimination of a latent fingerprint to retain a latent fingerprint pattern in an identification operation using a composition containing 2-cyanoacrylate, resulting in completion of the present invention.

That is, the present invention is a latent fingerprint detecting composition in which a 2-cyanoacrylate, a fluorescent dye and a silicone oil are included and a content of the silicone oil is in a range from 700 to 9000 parts by mass based on 100 parts by mass of the 2-cyanoacrylate.

The latent fingerprint detecting composition of the present invention may include further a stabilizer.

### [EFFECTS OF THE INVENTION]

According to the present invention, when a latent fingerprint detecting composition is brought into contact with a specimen, the composition is adsorbed to a fingertip secretory component forming a latent fingerprint and is cured to be whitened, without causing dissolution or migration of the fingertip secretory component forming the latent fingerprint or without causing dissolution, swelling or degeneration of the fingerprint support member. Therefore, a latent fingerprint pattern can be obtained with high accuracy. When a latent fingerprint detecting composition containing the fluorescent dye is used according to a color of the fingerprint support member, a whitened portion containing a fluorescent dye can be formed. Then, a light emission pattern reflecting the latent fingerprint can be obtained by irradiating the whitened portion with visible light or the like.

In the present invention, not only when a latent fingerprint containing a fingertip secretory component is formed on a surface of a flat fingerprint support member, but also when a latent fingerprint is formed only on the surfaces of projections on a fingerprint support member (e.g. porous member) having a surface with a fine protrusion and recess in sequence and, for example, a latent fingerprint pattern is discontinued, the fingerprint support member is not dissolved, swollen and degenerated by a silicone oil, and deformation or elimination of the remaining latent fingerprint is suppressed. Therefore, the latent fingerprint pattern is retained without depending on the surface shape of the fingerprint support member, and the latent fingerprint can be efficiently detected.

### [BRIEF DESCRIPTION OF THE DRAWINGS]

FIG. 1 is an external appearance image showing a brick after a surface thereof is touched with fingers, the brick being an evaluation sample of Example 1;
FIG. 2 is an image showing a fluorescent fingerprint obtained by bringing a composition in Example 1 into contact with a brick and then irradiating a latent fingerprint-bearing brick with visible light;
FIG. 3 is an external appearance image showing a back surface (magnetic surface) of a magnetic ticket sheet after a surface thereof is touched with fingers, the magnetic ticket sheet being an evaluation sample of Example 13;
FIG. 4 is an image showing a fluorescent fingerprint obtained by bringing a composition in Example 13 into contact with a magnetic ticket sheet and then irradiating a latent fingerprint-bearing magnetic ticket sheet with visible light;
FIG. 5 is an external appearance image showing a polyethylene bag after a surface thereof is touched, the polyethylene bag being an evaluation sample of Example 13; and
FIG. 6 is an image showing a fluorescent fingerprint obtained by bringing a composition in Example 13 into contact with a polyethylene bag and then irradiating a latent fingerprint-bearing polyethylene bag with visible light.

### [EMBODIMENTS FOR CARRYING OUT THE INVENTION]

Hereinafter, the present invention is described in detail.

The latent fingerprint detecting composition of the present invention is a liquid composition including a 2-cyanoacrylate, a fluorescent dye and a silicone oil in a specific content ratio.

The 2-cyanoacrylate is not particularly limited. A cyanoacrylic acid (cyclo)alkyl ester, a cyanoacrylic acid aryl ester, a cyanoacrylic acid alkenyl ester, a cyanoacrylic acid alkynyl ester, a cyanoacrylic acid alkoxyalkyl ester, a cyanoacrylic acid haloalkyl ester, a cyanoacrylic acid haloalkoxyalkyl ester, can be used. In the present invention, the 2-cyanoacrylate may be used singly or in combination of two or more types thereof.

Examples of the cyanoacrylic acid alkyl ester include methyl-2-cyanoacrylate, ethyl-2-cyanoacrylate, n-propyl-2-cyanoacrylate, isopropyl-2-cyanoacrylate, n-butyl-2-cyanoacrylate, isobutyl-2-cyanoacrylate, n-hexyl-2-cyanoacrylate, n-octyl-2-cyanoacrylate, 2-ethylhexyl-2-cyanoacrylate, cyclohexyl-2-cyanoacrylate. Among these, methyl-2-cyanoacrylate, ethyl-2-cyanoacrylate, n-propyl-2-cyanoacrylate, and isopropyl-2-cyanoacrylate are preferable.

Examples of the cyanoacrylic acid aryl ester include phenyl-2-cyanoacrylate, benzyl-2-cyanoacrylate,

Examples of the cyanoacrylic acid alkenyl ester include ally-2-cyanoacrylate.

Examples of the cyanoacrylic acid alkynyl ester include propargyl-2-cyanoacrylate.

Examples of the cyanoacrylic acid alkoxyalkyl ester include methoxymethyl-2-cyanoacrylate, methoxyethyl-2-cyanoacrylate, methoxybutyl-2-cyanoacrylate, ethoxyethyl-2-cyanoacrylate.

Examples of the cyanoacrylic acid haloalkoxyalkyl ester include 2-chloroethyl-2-cyanoacrylate, 2-chloroethoxyethyl-2-cyanoacrylate, 2,2,2-trifluoroethyl-2-cyanoacrylate.

The 2-cyanoacrylate is preferably a cyanoacrylic acid alkyl ester, and particularly preferably a cyanoacrylic acid alkyl ester having an alkyl portion having 1 to 3 carbon atoms.

The silicone oil is not particularly limited as long as it is liquid at 1 atm and at a temperature of 25°C, and any of a non-modified silicone oil and a modified silicone oil may be used. The silicone oil in the latent fingerprint detecting composition of the present invention may be consisting of one type or of two or more types thereof.

The silicone oil may be any of a chain compound or a cyclic compound.

Examples of the chain compound include an alkylsiloxane such as 1,1,3,3-tetramethyldisiloxane, 1,3 -diethyltetramethyldisiloxane, 1,1,3,3 -tetraisopropyldisiloxane, pentamethyldisiloxane, hexamethyldisiloxane, hexaethyldisiloxane, pentamethylhexyldisiloxane, 1,3-dioctyltetramethyldisiloxane, 1,1,1,5,5,5-hexamethyltrisiloxane, 1,1,3,3,5,5-hexamethyltrisiloxane, 1,1,1,3,5,5,5-heptamethyltrisiloxane, octamethyltrisiloxane, heptamethylhexyltrisiloxane, heptamethylheptyltrisiloxane, heptamethyloctyltrisiloxane, 1,1,3,3,5,5,7,7-octamethyltetrasiloxane, decamethyltetrasiloxane, dodecamethylpentasiloxane, tetradecamethylhexasiloxane, hexadecamethylheptasiloxane, octadecamethyloctasiloxane, eicosamethylnonasiloxane, docosamethyldecasiloxane, tris(trimethylsiloxy)silane, tris(trimethylsiloxymethyl)silane and tetrakis(trimethylsiloxy)silane; a modified alkylsiloxane such as hexavinyldisiloxane, (3-glycidoxypropyl)pentamethyldisiloxane, 1,1,3,3-tetraethoxy-1,3 -dimethyldisiloxane, 1,3 -bis(glycidoxypropyl)tetramethyldisiloxane, 1,3-bis(hydroxybutyl)tetramethyldisiloxane, 1,3-bis(hydroxypropyl)tetramethyldisiloxane, 1,3-bis(trimethylsiloxy)-1,3-dimethyldisiloxane, 1,3-diallyltetrakis(trimethylsiloxy)disiloxane, 1,3-diallyltetramethyldisiloxane, 1,3-diethyltetramethyldisiloxane, 1,3-diethynyltetramethyldisiloxane, 1,3-dimethyltetramethoxydisiloxane, 1,3-dioctyltetramethyldisiloxane, 1,3-divinyltetraethoxydisiloxane, 1,3-divinyltetramethyldisiloxane, 1-allyl-1,1,3,3 -tetramethyldisiloxane, divinyltetrakis(trimethylsiloxy)disiloxane, tris(trimethylsiloxyvinyl)silane, 1,5-divinylhexamethyltrisiloxane, 2-[methoxy(polyethyleneoxy)propyl]heptamethyltrisiloxane, 3-[hydroxy(polyethyleneoxy)propyl]heptamethyltrisiloxane and 3-octylheptamethyltrisiloxane.

Examples of the cyclic compound include a cycloalkylsiloxane such as hexamethylcyclotrisiloxane, octamethylcyclotetrasiloxane, decamethylcyclopentasiloxane, dodecamethylcyclohexasiloxane, tetradecamethylcycloheptasiloxane, hexadecamethylcyclooctasiloxane, octadecamethylcyclononasiloxane and eicosamethylcyclodecasiloxane; a modified cycloalkylsiloxane such as bis[(bicycloheptenyl)ethyl]tetramethyldisiloxane and bis-2-[3,4-(epoxycyclohexyl)ethyl]tetramethyldisiloxane.

The silicone oil is preferably an alkylsiloxane, more preferably an alkylsiloxane having 2 to 10 silicon atoms, and particularly preferably an alkylsiloxane having 2 to 5 silicon atoms, because without depending on a constituent material and surface shape of a fingerprint support member, deformation or elimination of the latent fingerprint is suppressed, a latent fingerprint pattern is retained, and a stable whitened portion is formed on the latent fingerprint, so that the latent fingerprint can be easily detected. Since a method for bringing the latent fingerprint detecting composition of the present invention into contact with a specimen may include spraying, immersion or the like, as is described later, a composition having a viscosity corresponding to the properties of the specimen can be used. Thus, two or more alkylsiloxanes having 2 to 5 silicon atoms, or a combination of an alkylsiloxane having 2 to 5 silicon atoms and an alkylsiloxane having 6 or more silicon atoms or other silicone oil having a high viscosity can be used. In addition, the silicone oil is a component in which the content ratio thereof is high in the composition. Therefore when the composition contains a silicone oil having a boiling point ranging preferably from 90°C to 300°C, and more preferably from 90°C to 250°C at a pressure of 1 atm, the latent fingerprint can be detected more quickly.

A content of the silicone oil in the latent fingerprint detecting composition of the present invention is in a range from 700 to 9000 parts by mass, preferably from 1000 to 5000 parts by mass, and more preferably from 1500 to 4500 parts by mass based on 100 parts by mass of the content of 2-cycanoacrylate for ensuring that deformation or elimination of the latent fingerprint is suppressed, a latent fingerprint pattern is retained, and a stable whitened portion is formed on the latent fingerprint, so that the latent fingerprint can be easily detected.

The latent fingerprint detecting composition of the present invention may further contain other components in addition to the above-mentioned essential components. The latent fingerprint detecting composition may include a stabilizer, a thickener, a thixotropy imparting agent, a flavor.

A cured portion formed by polymerization of the 2-cyanoacrylate is normally white, and therefore a latent fingerprint detecting composition containing a fluorescent dye is used depending on the color of the fingerprint support member.

The fluorescent dye is not particularly limited as long as a sample obtained by dispersing a powder of the fluorescent dye in a powder composed of a homopolymer of the 2-cyanoacrylate emits fluorescence when irradiated with an electromagnetic wave (visible light) having a wavelength ranging from 400 to 800 nm. In the present invention, an anthraquinone-based dye, a naphthalimide-based dye and the like can be used, because they are stable in a polymer of the 2-cyanoacrylate and have a high fluorescence sensitivity. The fluorescent dye in the latent fingerprint detecting composition of the present invention may be one type or a combination of two or more types thereof.

Examples of the anthraquinone-based dye include an aminoanthraquinone, an aminohydroxyanthraquinone, a diaminoanthraquinone, a dihydroxyanthraquinone, a diaminodihydroxyanthraquinone. Among them, an aminohydroxyanthraquinone such as 2-phenoxy-1-amino-4-hydroxyanthracene-9,10-dione (SOLVENT RED 146) is preferable.

Examples of the naphthalimide-based dye include an alkylnaphthalimide, an alkoxynaphthalimide, an alkoxyalkylnaphthalimide, an aminonaphthalimide, an alkylaminaphthalimide, a nitronaphthalimide, a halogenated naphthalimide, a carbonylnaphthalimide, a phenylthionaphthalimide, a cyanonaphthalimide, a hydroxynaphthalimide. Among them, an alkylaminaphthalimide such as 2-butyl-6-(butylamino)-1H-benz[de]isoquinoline-1,3(2H)-dione (SOLVENT YELLOW 43) is preferable.

Solubility of the fluorescent dye in the silicone oil is not particularly limited. It is preferable to use a fluorescent dye soluble in the silicone oil. When a fluorescent dye insoluble in the silicone oil is used, the average particle size thereof is preferably 0.1 µm or smaller.

The latent fingerprint detecting composition of the present invention contains a fluorescent dye; a content thereof is preferably 0.05 part or more by mass, more preferably in a range from 0.1 to 6.0 parts by mass, further preferably from 0.2 to 3.0 parts by mass, and particularly preferably from 0.3 to 1.0 part by mass based on 100 parts by mass of the 2-cyanoacrylate for ensuring that a sufficient light emission intensity is obtained by photoirradiation, so that the latent fingerprint can be easily detected.

The stabilizer has an action of suppressing polymerization of the 2-cyanoacrylate in the composition. A sulfurous acid gas, methanesulfonic acid, ethanesulfonic acid, HBF₄, BF₃ ether complex salts, BF₃ acetate complex salts, hydroquinone, hydroquinone monomethyl ether, 2,6-di-tert-butyl-p-cresol, trifluoromethanesulfonic acid, carbon disulfide can be used.

In a case where the latent fingerprint detecting composition of the present invention contains a stabilizer, a content thereof is in a range from 0.0001 to 1 part by mass, preferably from 0.001 to 0.1 part by mass, and more preferably from 0.001 to 0.05 part by mass based on 100 parts by mass of a content of the 2-cyanoacrylate.

The latent fingerprint detecting composition of the present invention may be a homogeneous solution or a homogeneous dispersion liquid. A viscosity thereof is preferably in a range from 0.5 to 20 mPa•s, and more preferably from 0.5 to 10 mPa•s. The viscosity of the composition is measured at a temperature of 25°C by a method in accordance with "Method 2" in JIS K 6833-1 5.4.1b).

The latent fingerprint detecting composition of the present invention can be produced by mixing the above-mentioned essential components or by mixing the above-mentioned essential components with other components. When a stabilizer is used, it is preferable to form a mixture of the stabilizer with the 2-cyanoacrylate beforehand. When the composition is produced, a method for mixing raw materials (e.g. a method for using raw materials) can be selected according to, for example, a type of the fluorescent dye. All the raw materials may be mixed at once, or dividedly mixed.

According to the latent fingerprint detecting composition of the present invention, detection of the latent fingerprint can be efficiently conducted without being limited by a type of a constituent material of the fingerprint support member. That is, a latent fingerprint formed on a surface of the fingerprint support member composed of any of an inorganic material such as a metal, an alloy and an inorganic compound, an organic material such as a resin, or a composite material containing the foregoing materials can be detected. In addition, a surface shape of the fingerprint support member is not limited, and the latent fingerprint detecting composition of the present invention is particularly suitable for a fingerprint support member which has a surface provided with a fine projection and recess in sequence and which has been heretofore difficult to detect by a known latent fingerprint detecting composition, including, for example, a porous member such as a brick, concrete block, plastic sintered porous body, slate, wood and paper, and a back surface of a magnetic paper such as a ticket sheet.

As described above, a cured portion formed by polymerization of the 2-cyanoacrylate is normally white. Therefore it is preferable that a latent fingerprint detecting composition containing a fluorescent dye that emits light having a color different from the color of the fingerprint support member is used depending on the color of the fingerprint support member.

When the latent fingerprint detecting composition of the present invention is brought into contact with a specimen, the composition is adsorbed to a latent fingerprint containing water and a lipid, and the 2-cyanoacrylate is polymerized concurrently with the contact or during drying to form a whitened portion on a surface of the latent fingerprint. On the other hand, in a region having no latent fingerprint, the composition is not adsorbed, and also erosion or deformation does not occur. Therefore, a latent fingerprint pattern can be efficiently detected. When a latent fingerprint detecting composition containing a fluorescent dye that emits light having a color different from the color of the fingerprint support member when irradiated with light is used, a light emission pattern reflecting a latent fingerprint can be obtained by irradiating the whitened portion with light having a wavelength ranging from 400 to 800 nm.

Examples of the method for bringing the latent fingerprint detecting composition of the present invention into contact with a specimen include a spraying method (spray method), and an immersion method.

In the present invention, the spraying method is preferable because it is not only capable of reducing the use amount of the composition for detecting the latent fingerprint, but also suitable for a specimen having a large area.

When the composition is sprayed to a specimen, a sprayer such as a mechanical sprayer, an electric sprayer, a pump-type spray, a trigger-type spray and an aerosol can be used. A spraying number to the same surface of the specimen is not particularly limited as long as the spraying amount of the 2-cyanoacrylate is preferably in a range from 0.000015 to 0.003 ml/cm². The spraying amount per one spray is preferably in a range from 0.0005 to 1 ml/cm², and more preferably from 0.001 to 0.1 ml/cm².

When a specimen is immersed in the composition, it is preferable that the specimen is immersed for 0.1 to 5 minutes with the specimen made stationary in the composition of which temperature is in a range from 0°C to 40°C.

### [EXAMPLES]

Hereinafter, the present invention is specifically described using Examples. The present invention is not limited to the following Examples.

### 1. Raw materials for latent fingerprint detecting composition

Raw materials used for producing a latent fingerprint detecting composition are as follows.

### 1-1. 2-cyanoacrylate

Ethyl-2-cyanoacrylate with 200 ppm by mass of sulfur dioxide gas and 600 ppm by mass of hydroquinone as stabilizers added thereto beforehand (hereinafter, referred to as "ethyl-2-cyanoacrylate raw material") was used.

### 1-2. Fluorescent dye

The following two products subjected to a purification treatment were used.

### (1) Anthraquinone-based dye

2-phenoxy-1-amino-4-hydroxyanthracene-9,10-dione "Kayaset Red B" (trade name) manufactured by Nippon Kayaku Co., Ltd.

### (2) Naphthalimide-based dyes

2-butyl-6-(butylamino)-1H-benz[de]isoquinoline-1,3(2H)-dione "Kayaset Flavine FG" (trade name) manufactured by Nippon Kayaku Co. Ltd.

### <Purification method>

5g of a fluorescent dye was gradually added to 50g of a methanol solution of 1% by mass of methanesulfonic acid under stirring, and after completion of addition, the stirring was continued for further 5 minutes. The mixture was then separated by filtration, and dried under reduced pressure at a temperature of 50°C for 24 hours.

### 1-3. Silicone oil

(1) Hexamethyldisiloxane (boiling point at 1 atm: 100°C)
(2) Decamethyltetrasiloxane (boiling point at 1 atm: 194°C)
(3) Decamethylcyclopentasiloxane (boiling point at 1 atm: 210°C)

### 1-4. Stabilizer

A sulfurous acid gas and hydroquinone were used.

### 1-5. Organic solvent

For Comparative Examples, acetone, methylethylketone, benzene, hexane and chloroform were used in place of the silicone oil.

### 2. Production and evaluation of latent fingerprint detecting composition

The above-mentioned raw materials were dividedly mixed, and stirred at a temperature of 25°C to prepare a latent fingerprint detecting composition. The viscosity of the resulting composition was measured at a temperature of 25°C by a method in accordance with "Method 2" in JIS K 6833-1 5.4.1b).

A surface of a commercially available brick, a surface of a commercially available polyethylene bag and a back surface (magnetic surface) of a magnetic ticket sheet, which were pressed with the pad of a finger after washing of hands, were provided as specimens. The composition was sprayed to the whole surface of each of the specimens, and whether or not it was possible to detect a latent fingerprint by photoirradiation was examined.

### 2-1. Detection of latent fingerprint on brick surface

### Example 1

A homogeneous solution (latent fingerprint detecting composition) containing 3.7 parts by mass of the ethyl-2-cyanoacrylate raw material, 96.3 parts by mass of hexamethyldisiloxane and 281.3 × 10⁻⁶ part by mass of the anthraquinone-based dye was obtained. The viscosity (25°C) was 0.65 mPa•s.

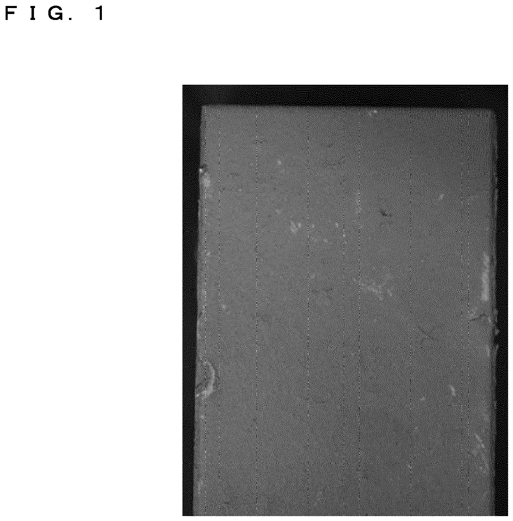

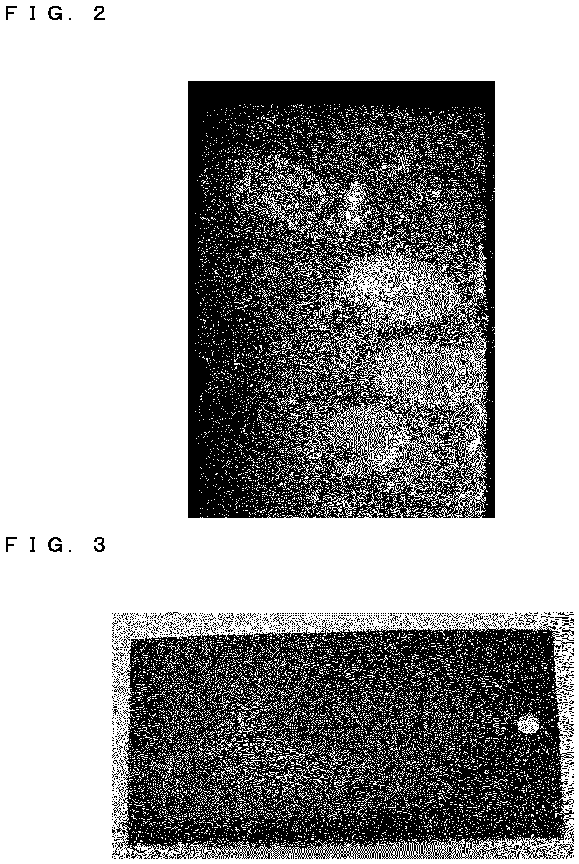

Subsequently, the resulting latent fingerprint detecting composition was sprayed (spraying amount: 0.01 to 0.02 ml/cm²) to a brick (FIG. 1) having a latent fingerprint on a surface. When the sprayed composition was coming into contact with a fingertip secretory component, a whitening phenomenon by curing of ethyl-2-cyanoacrylate was observed. Thereafter, the sprayed composition was dried. A forensic light source device "CRIMESCOPE CS-16-500" (trade name) manufactured by Horiba Scientific Co., Ltd., was used and a white solidified portion was irradiated with light having a wavelength ranging from 415 to 575 nm. As a result, clear light emission for the latent fingerprint was observed as shown in FIG. 2. The results thereof are shown in Table 1.

### Example 2

A homogeneous solution (latent fingerprint detecting composition) containing 5.5 parts by mass of the ethyl-2-cyanoacrylate raw material, 94.5 parts by mass of hexamethyldisiloxane and 368.1 × 10⁻⁶ part by mass of the anthraquinone-based dye was obtained. The viscosity (25°C) was 0.65 mPa•s.

After that, the same procedure as in Example 1 was carried out and clear light emission in the latent fingerprint by photoirradiation was observed. The results thereof are shown in Table 1.

### Example 3

A homogeneous solution (latent fingerprint detecting composition) containing 5.5 parts by mass of the ethyl-2-cyanoacrylate raw material, 94.5 parts by mass of hexamethyldisiloxane and 36.8 × 10⁻⁶ part by mass of the anthraquinone-based dye was obtained. The viscosity (25°C) was 0.65 mPa•s.

After that, the same spraying as in Example 1 was carried out seven times, followed by performing photoirradiation, and as a result, clear light emission in the latent fingerprint was observed. The results thereof are shown in Table 1. Since the latent fingerprint was detected after the composition was sprayed multiple times, the sample was rated "Δ".

### Example 4

A homogeneous solution (latent fingerprint detecting composition) containing 3.7 parts by mass of the ethyl-2-cyanoacrylate raw material, 96.3 parts by mass of octamethyltrisiloxane and 281.3 × 10⁻⁶ part by mass of the anthraquinone-based dye was obtained. The viscosity (25°C) was 1.0 mPa•s.

After that, the same procedure as in Example 1 was carried out and clear light emission in the latent fingerprint by photoirradiation was observed. The results thereof are shown in Table 1.

### Example 5

A homogeneous solution (latent fingerprint detecting composition) containing 5.5 parts by mass of the ethyl-2-cyanoacrylate raw material, 94.5 parts by mass of octamethyltrisiloxane and 368.1 × 10⁻⁶ part by mass of the anthraquinone-based dye was obtained. The viscosity (25°C) was 1.0 mPa•s.

After that, the same procedure as in Example 1 was carried out and clear light emission in the latent fingerprint by photoirradiation was observed. The results thereof are shown in Table 1.

### Example 6

A homogeneous solution (latent fingerprint detecting composition) containing 5.5 parts by mass of the ethyl-2-cyanoacrylate raw material, 94.5 parts by mass of decamethyltetrasiloxane and 368.1 × 10⁻⁶ part by mass of the anthraquinone-based dye was obtained. The viscosity (25°C) was 1.5 mPa•s.

After that, the same procedure as in Example 1 was carried out and clear light emission in the latent fingerprint by photoirradiation was observed. The results thereof are shown in Table 1.

### Example 7

A homogeneous solution (latent fingerprint detecting composition) containing 5.5 parts by mass of the ethyl-2-cyanoacrylate raw material, 94.5 parts by mass of decamethylcyclopentasiloxane and 368.1 × 10⁻⁶ part by mass of the anthraquinone-based dye was obtained. The viscosity (25°C) was 4.0 mPa•s.

After that, the same procedure as in Example 1 was carried out and clear light emission in the latent fingerprint by photoirradiation was observed. The results thereof are shown in Table 1.

### Example 8

A homogeneous solution (latent fingerprint detecting composition) containing 3.7 parts by mass of the ethyl-2-cyanoacrylate raw material, 96.3 parts by mass of hexamethyldisiloxane and 140.6 × 10⁻⁶ part by mass of the naphthalimide-based dye was obtained. The viscosity (25°C) was 0.65 mPa•s.

After that, the same procedure as in Example 1 was carried out and clear light emission in the latent fingerprint by photoirradiation was observed. The results thereof are shown in Table 1.

### Example 9

A homogeneous solution (latent fingerprint detecting composition) containing 5.5 parts by mass of the ethyl-2-cyanoacrylate raw material, 94.5 parts by mass of hexamethyldisiloxane and 184.0 × 10⁻⁶ parts by mass of the naphthalimide-based dye was obtained. The viscosity (25°C) was 0.65 mPa•s.

After that, the same procedure as in Example 1 was carried out and clear light emission in the latent fingerprint by photoirradiation was observed. The results thereof are shown in Table 1.

### Example 10

A homogeneous solution (latent fingerprint detecting composition) containing 5.5 parts by mass of the ethyl-2-cyanoacrylate raw material, 94.5 parts by mass of hexamethyldisiloxane and 18.4 × 10⁻⁶ part by mass of the naphthalimide-based dye was obtained. The viscosity (25°C) was 0.65 mPa•s.

After that, the same spraying as in Example 1 was carried out five times, followed by performing photoirradiation, and as a result, clear light emission in the latent fingerprint was observed. The results thereof are shown in Table 1. Since the latent fingerprint was detected after the composition was sprayed multiple times, the sample was rated "Δ".

### Example 11

A homogeneous solution (latent fingerprint detecting composition) containing 3.7 parts by mass of the ethyl-2-cyanoacrylate raw material, 96.3 parts by mass of octamethyltrisiloxane and 140.6 × 10⁻⁶ part by mass of the naphthalimide-based dye was obtained. The viscosity (25°C) was 1.0 mPa•s.

After that, the same procedure as in Example 1 was carried out and clear light emission in the latent fingerprint by photoirradiation was observed. The results thereof are shown in Table 1.

### Example 12

A homogeneous solution (latent fingerprint detecting composition) containing 5.5 parts by mass of the ethyl-2-cyanoacrylate raw material, 94.5 parts by mass of octamethyltrisiloxane and 184.0 × 10⁻⁶ part by mass of the naphthalimide-based dye was obtained. The viscosity (25°C) was 1.0 mPa•s.

After that, the same procedure as in Example 1 was carried out and clear light emission in the latent fingerprint by photoirradiation was observed. The results thereof are shown in Table 1.

### Comparative Example 1

A homogeneous solution (latent fingerprint detecting composition) containing 5.5 parts by mass of the ethyl-2-cyanoacrylate raw material, 94.5 parts by mass of acetone and 368.1 × 10⁻⁶ part by mass of the anthraquinone-based dye was obtained. The viscosity (25°C) was 0.34 mPa•s.

After that, the same procedure as in Example 1 was carried out. Light emission by photoirradiation was not observed, and thus it was not possible to detect the latent fingerprint. The results thereof are shown in Table 1.

### Comparative Example 2

A homogeneous solution (latent fingerprint detecting composition) containing 5.5 parts by mass of the ethyl-2-cyanoacrylate raw material, 94.5 parts by mass of methylethylketone and 368.1 × 10⁻⁶ part by mass of the anthraquinone-based dye was obtained. The viscosity (25°C) was 0.4 mPa•s.

After that, the same procedure as in Example 1 was carried out. Light emission by photoirradiation was not observed, and thus it was not possible to detect the latent fingerprint. The results thereof are shown in Table 1.

### Comparative Example 3

A homogeneous solution (latent fingerprint detecting composition) containing 5.5 parts by mass of the ethyl-2-cyanoacrylate raw material, 94.5 parts by mass of benzene and 368.1 × 10⁻⁶ part by mass of the anthraquinone-based dye was obtained. The viscosity (25°C) was 0.65 mPa•s.

After that, the same procedure as in Example 1 was carried out. Light emission by photoirradiation was not observed, and thus it was not possible to detect the latent fingerprint. The results thereof are shown in Table 1.

### Comparative Example 4

A homogeneous solution (latent fingerprint detecting composition) containing 5.5 parts by mass of the ethyl-2-cyanoacrylate raw material, 94.5 parts by mass of hexane and 368.1 × 10⁻⁶ part by mass of the anthraquinone-based dye was obtained. The viscosity (25°C) was 0.33 mPa•s.

After that, the same procedure as in Example 1 was carried out. Light emission by photoirradiation was not observed, and thus it was not possible to detect the latent fingerprint. The results thereof are shown in Table 1.

### Comparative Example 5

A homogeneous solution (latent fingerprint detecting composition) containing 5.5 parts by mass of the ethyl-2-cyanoacrylate raw material, 94.5 parts by mass of chloroform and 368.1 × 10⁻⁶ part by mass of the anthraquinone-based dye was obtained. The viscosity (25°C) was 0.57 mPa•s.

After that, the same procedure as in Example 1 was carried out. Light emission by photoirradiation was not observed, and thus it was not possible to detect the latent fingerprint. The results thereof are shown in Table 1.

**Table 1**

| | Latent fingerprint detecting composition | | | | | | | | Evaluation |
|---|---|---|---|---|---|---|---|---|---|
| | Silicone oil (parts by mass) | | | | Organic solvent (parts by mass) | 2-Cyanoacrylate (parts by mass) | Fluorescent dye (parts by mass) | | Brick |
| | Hexamethyl disiloxane | Octamethyl trisiloxane | Decamethyl tetrasiloxane | Decamethyl cyclopentasiloxane | | | Anthraquinone-based | Naphthalimide-based | |
| Example 1 | 96.3(2602.7) | | | | | 3.7(100) | 281.3×10⁻⁶(0.76) | | ○ |
| Example 2 | 94.5(1718.2) | | | | | 5.5(100) | 368.1×10⁻⁶(0.67) | | ○ |
| Example 3 | 94.5(1718.2) | | | | | 5.5(100) | 36.8×10⁻⁶(0.07) | | Δ |
| Example 4 | | 96.3(2602.7) | | | | 3.7(100) | 281.3×10⁻⁶(0.76) | | ○ |
| Example 5 | | 94.5(1718.2) | | | | 5.5(100) | 368.1×10⁻⁶(0.67) | | ○ |
| Example 6 | | | 94.5(1718.2) | | | 5.5(100) | 3681×10⁻⁶(0.67) | | ○ |
| Example 7 | | | | 94.5(1718.2) | | 5.5(100) | 368.1×10⁻⁶(0.67) | | ○ |
| Comparative example 1 | | | | | Acetone 94.5(1718.2) | 5.5(100) | 368.1×10⁻⁶(0.67) | | × |
| Comparative example 2 | | | | | Methylethylketone 94.5(1718..2) | 5.5(100) | 368.1×10⁻⁶(0.67) | | × |
| Comparative example 3 | | | | | Benzene 94.5(1718.2) | 5.5(100) | 368.1×10⁻⁶(0.67) | | × |
| Comparative example 4 | | | | | Hexane 94.5(1718.2) | 5.5(100) | 368.1×10⁻⁶(0.67) | | × |
| Comparative example 5 | | | | | Chlorofolm 94.5(1718.2) | 5.5(100) | 368.1×10⁻⁶(0.67) | | × |
| Example 8 | 96.3(2602.7) | | | | | 3.7(100) | | 140.6×10⁻⁶(0.38) | ○ |
| Example 9 | 94.5(1718.2) | | | | | 5.5(100) | | 184.0×10⁻⁶(0.33) | ○ |
| Example 10 | 94.5(1718.2) | | | | | 5.5(100) | | 36.8×10⁻⁶(0.07) | Δ |
| Example 11 | | 96.3(2602.7) | | | | 3.7(100) | | 140.6x×10⁻⁶(0.38) | ○ |
| Example 12 | | 94.5(1718.2) | | | | 5.5(100) | | 184.0×10⁻⁶(0.33) | ○ |

| | | | | | | | | | |
|---|---|---|---|---|---|---|---|---|---|
| (Note) The parenthesized value in the table is a ratio based on 100 parts by mass of 2-cyanoacrylate. | | | | | | | | | |

### 2-2. Detection of latent fingerprint on back surface (magnetic surface) of magnetic ticket sheet or surface of polyethylene bag

### Example 13

A homogeneous solution (latent fingerprint detecting composition) containing 2.3 parts by mass of the ethyl-2-cyanoacrylate raw material, 97.7 parts by mass of hexamethyldisiloxane and 133.2 × 10⁻⁶ part by mass of the anthraquinone-based dye was obtained. The viscosity (25°C) was 0.65 mPa•s.

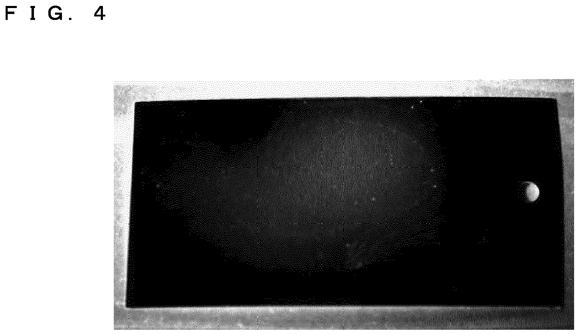

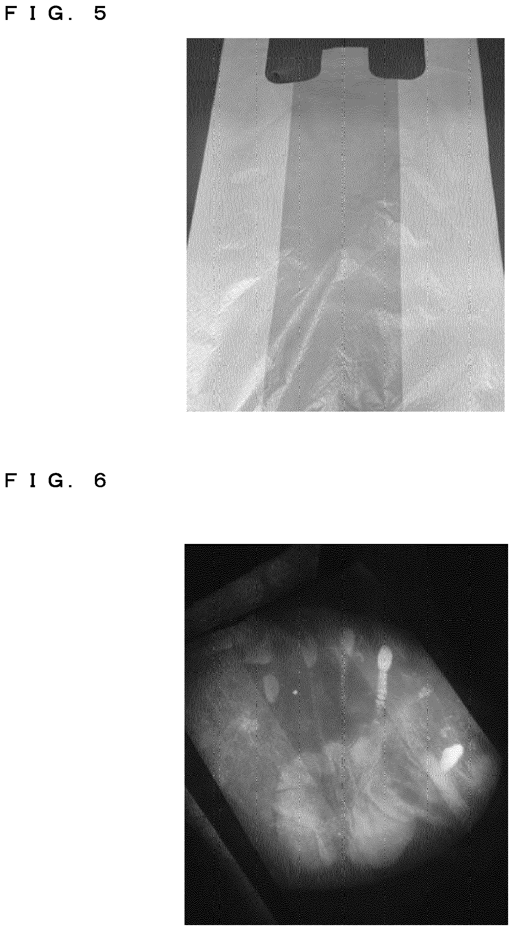

Subsequently, the resulting latent fingerprint detecting composition was sprayed (spraying amount: 0.01 to 0.02 ml/cm²) to a magnetic surface of a magnetic ticket sheet (FIG. 3) and a surface of a polyethylene bag (FIG. 5), each of which had a latent fingerprint. When the sprayed composition was coming into contact with a fingertip secretory component, a whitening phenomenon by curing of ethyl-2-cyanoacrylate was observed. Thereafter, the sprayed composition was dried. A forensic light source device "CRIMESCOPE CS-16-500" (trade name) manufactured by Horiba Scientific Co., Ltd., was used and a white solidified portion was irradiated with light having a wavelength ranging from 415 to 575 nm. As a result, clear light emissions for the latent fingerprint were observed as shown in FIGs. 4 and 6. The results thereof are shown in Table 2.

### Example 14

A homogeneous solution (latent fingerprint detecting composition) containing 3.7 parts by mass of the ethyl-2-cyanoacrylate raw material, 96.3 parts by mass of hexamethyldisiloxane and 187.5 × 10⁻⁶ part by mass of the anthraquinone-based dye was obtained. The viscosity (25°C) was 0.65 mPa•s.

After that, the same procedure as in Example 13 for a polyethylene bag having a latent fingerprint on a surface thereof was carried out and clear light emission in the latent fingerprint by photoirradiation was observed. The results thereof are shown in Table 2.

### Example 15

A homogeneous solution (latent fingerprint detecting composition) containing 3.7 parts by mass of the ethyl-2-cyanoacrylate raw material, 96.3 parts by mass of hexamethyldisiloxane and 18.8 × 10⁻⁶ part by mass of the anthraquinone-based dye was obtained. The viscosity (25°C) was 0.65 mPa•s.

After that, the same spraying as in Example 13 for a polyethylene bag having a latent fingerprint on a surface thereof was carried out three times, followed by performing photoirradiation, and as a result, clear light emission in the latent fingerprint was observed. The results thereof are shown in Table 2. Since the latent fingerprint was detected after the composition was sprayed multiple times, the sample was rated "Δ".

### Example 16

A homogeneous solution (latent fingerprint detecting composition) containing 2.3 parts by mass of the ethyl-2-cyanoacrylate raw material, 97.7 parts by mass of octamethyltrisiloxane and 133.2 × 10⁻⁶ part by mass of the anthraquinone-based dye was obtained. The viscosity (25°C) was 1.0 mPa•s.

After that, the same procedure as in Example 13 for a polyethylene bag having a latent fingerprint on a surface thereof was carried out and clear light emission in the latent fingerprint by photoirradiation was observed. The results thereof are shown in Table 2.

### Example 17

A homogeneous solution (latent fingerprint detecting composition) containing 3.7 parts by mass of the ethyl-2-cyanoacrylate raw material, 96.3 parts by mass of octamethyltrisiloxane and 187.5 × 10⁻⁶ part by mass of the anthraquinone-based dye was obtained. The viscosity (25°C) was 1.0 mPa•s.

After that, the same procedure as in Example 13 for a polyethylene bag having a latent fingerprint on a surface thereof was carried out and clear light emission in the latent fingerprint by photoirradiation was observed. The results thereof are shown in Table 2.

### Example 18

A homogeneous solution (latent fingerprint detecting composition) containing 3.7 parts by mass of the ethyl-2-cyanoacrylate raw material, 96.3 parts by mass of decamethyltetrasiloxane and 187.5 × 10⁻⁶ part by mass of the anthraquinone-based dye was obtained. The viscosity (25°C) was 1.5 mPa•s.

After that, the same procedure as in Example 13 for a polyethylene bag having a latent fingerprint on a surface thereof was carried out and clear light emission in the latent fingerprint by photoirradiation was observed. The results thereof are shown in Table 2.

### Example 19

A homogeneous solution (latent fingerprint detecting composition) containing 3.7 parts by mass of the ethyl-2-cyanoacrylate raw material, 96.3 parts by mass of decamethylcyclopentasiloxane and 187.5 × 10⁻⁶ part by mass of the anthraquinone-based dye was obtained. The viscosity (25°C) was 4.0 mPa•s.

After that, the same procedure as in Example 13 for a polyethylene bag having a latent fingerprint on a surface thereof was carried out and clear light emission in the latent fingerprint by photoirradiation was observed. The results thereof are shown in Table 2.

### Example 20

A homogeneous solution (latent fingerprint detecting composition) containing 2.3 parts by mass of the ethyl-2-cyanoacrylate raw material, 97.7 parts by mass of hexamethyldisiloxane and 66.6 × 10⁻⁶ part by mass of the naphthalimide-based dye was obtained. The viscosity (25°C) was 0.65 mPa•s.

After that, the same procedure as in Example 13 for a polyethylene bag having a latent fingerprint on a surface thereof was carried out and clear light emission in the latent fingerprint by photoirradiation was observed. The results thereof are shown in Table 2.

### Example 21

A homogeneous solution (latent fingerprint detecting composition) containing 3.7 parts by mass of the ethyl-2-cyanoacrylate raw material, 96.3 parts by mass of hexamethyldisiloxane and 93.8 × 10⁻⁶ part by mass of the naphthalimide-based dye was obtained. The viscosity (25°C) was 0.65 mPa•s.

After that, the same procedure as in Example 13 for a polyethylene bag having a latent fingerprint on a surface thereof was carried out and clear light emission in the latent fingerprint by photoirradiation was observed. The results thereof are shown in Table 2.

### Example 22

A homogeneous solution (latent fingerprint detecting composition) containing 3.7 parts by mass of the ethyl-2-cyanoacrylate raw material, 96.3 parts by mass of hexamethyldisiloxane and 9.4 × 10⁻⁶ part by mass of the naphthalimide-based dye was obtained. The viscosity (25°C) was 0.65 mPa•s.

After that, the same spraying as in Example 13 for a polyethylene bag having a latent fingerprint on a surface thereof was carried out five times, followed by performing photoirradiation, and as a result, clear light emission in the latent fingerprint was observed. The results thereof are shown in Table 2. Since the latent fingerprint was detected after the composition was sprayed multiple times, the sample was rated "Δ".

### Example 23

A homogeneous solution (latent fingerprint detecting composition) containing 2.3 parts by mass of the ethyl-2-cyanoacrylate raw material, 97.7 parts by mass of octamethyltrisiloxane and 66.6 × 10⁻⁶ part by mass of the naphthalimide-based dye was obtained. The viscosity (25°C) was 1.0 mPa•s.

After that, the same procedure as in Example 13 for a polyethylene bag having a latent fingerprint on a surface thereof was carried out and clear light emission in the latent fingerprint by photoirradiation was observed. The results thereof are shown in Table 2.

### Example 24

A homogeneous solution (latent fingerprint detecting composition) containing 3.7 parts by mass of the ethyl-2-cyanoacrylate raw material, 96.3 parts by mass of octamethyltrisiloxane and 93.8 × 10⁻⁶ part by mass of the naphthalimide-based dye was obtained. The viscosity (25°C) was 1.0 mPa•s.

After that, the same procedure as in Example 13 for a polyethylene bag having a latent fingerprint on a surface thereof was carried out and clear light emission in the latent fingerprint by photoirradiation was observed. The results thereof are shown in Table 2.

### Comparative Example 6

A homogeneous solution (latent fingerprint detecting composition) containing 3.7 parts by mass of the ethyl-2-cyanoacrylate raw material, 96.3 parts by mass of acetone and 187.5 × 10⁻⁶ part by mass of the anthraquinone-based dye was obtained. The viscosity (25°C) was 0.34 mPa•s.

After that, the same procedure as in Example 13 for a polyethylene bag having a latent fingerprint on a surface thereof was carried out. Light emission by photoirradiation was not observed, and thus it was not possible to detect the latent fingerprint. The results thereof are shown in Table 2.

### Comparative Example 7

A homogeneous solution (latent fingerprint detecting composition) containing 3.7 parts by mass of the ethyl-2-cyanoacrylate raw material, 96.3 parts by mass of methylethylketone and 187.5 × 10⁻⁶ part by mass of the anthraquinone-based dye was obtained. The viscosity (25°C) was 0.4 mPa•s.

After that, the same procedure as in Example 13 for a polyethylene bag having a latent fingerprint on a surface thereof was carried out. Light emission by photoirradiation was not observed, and thus it was not possible to detect the latent fingerprint. The results thereof are shown in Table 2.

### Comparative Example 8

A homogeneous solution (latent fingerprint detecting composition) containing 3.7 parts by mass of the ethyl-2-cyanoacrylate raw material, 96.3 parts by mass of benzene and 187.5 × 10⁻⁶ part by mass of the anthraquinone-based dye was obtained. The viscosity (25°C) was 0.65 mPa•s.

After that, the same procedure as in Example 13 for a polyethylene bag having a latent fingerprint on a surface thereof was carried out. Light emission by photoirradiation was not observed, and thus it was not possible to detect the latent fingerprint. The results thereof are shown in Table 2.

**Table 2**

| | Latent fingerprint detecting composition | | | | | | | | Evaluation | |
|---|---|---|---|---|---|---|---|---|---|---|
| | Silicone oil (parts by mass) | | | | Organic solvent (parts by mass) | 2-Cyanoacrylate (parts by mass) | Fluorescent dye (parts by mass) | | Polyethylene bag | Ticket sheet |
| | Hexamethyl disiloxane | Octamethyl trisiloxane | Decamethyl tetrasiloxane | Decamethyl cyclopentasiloxane | | | Anthraquinone-based | Naphthalimide-based | | |
| Example 13 | 97.7(4247.8) | | | | | 2.3(100) | 133.2×10⁻⁶ (0.58) | | ○ | ○ |
| Example 14 | 96.3(2602.7) | | | | | 3.7(100) | 187.5×10⁻⁶(0.51) | | ○ | - |
| Example 15 | 96.3(2602.7) | | | | | 3.7(100) | 18.8×10⁻⁶(0.05) | | Δ | |
| Example 16 | | 97.7(4247.8) | | | | 2.3(100) | 133.2×10⁻⁶(0.58) | | ○ | - |
| Example 17 | | 96.3(2602.7) | | | | 3.7(100) | 187.5×10⁻⁶ (0.51) | | ○ | - |
| Example 18 | | | 96.3(2602.7) | | | 3.7(100) | 187.5×10⁻⁶ (0.51) | | ○ | - |
| Example 19 | | | | 96.3(2602.7) | | 3.7(100) | 187.5×10⁻⁶(0.51) | | ○ | - |
| Comparative example 6 | | | | | Acetone 96.3(2602.7) | 3.7(100) | 187.5×10⁻⁶(0.51) | | × | - |
| Comparative example 7 | | | | | Methylethylketone 96.3(2602.7) | 3.7(100) | 187.5×10⁻⁶(0.51) | | × | - |
| Comparative example 8 | | | | | Benzene 96.3(2602.7) | 3.7(100) | 187.5×10⁻⁶(0.51) | | × | - |
| Example 20 | 97.7(4247.8) | | | | | 2.3(100) | | 66.6×10⁻⁶(0.29) | ○ | - |
| Example 21 | 96.3(2602.7) | | | | | 3.7(100) | | 938×10⁻⁶(0.25) | ○ | - |
| Example 22 | 96.3(2602.7) | | | | | 3.7(100) | | 9.4×10⁻⁶(0.03) | Δ | |
| Example 23 | | 97.7(4247.8) | | | | 2.3(100) | | 66.6×10⁻⁶(0.29) | ○ | - |
| Example 24 | | 96.3(2602.7) | | | | 3.7(100) | | 93.8×10⁻⁶(0.25) | ○ | - |

| | | | | | | | | | | |
|---|---|---|---|---|---|---|---|---|---|---|
| (Note) The parenthesized value in the table is a ratio based on 100 parts by mass of 2-cyanoacrylate. | | | | | | | | | | |

### [INDUSTRIAL APPLICABILITY]

According to the present invention, a latent fingerprint can be accurately detected by bringing a latent fingerprint detecting composition into contact with a surface of a specimen, and then conducting photoirradiation. In particular, when the latent fingerprint is formed on a projection surface of a fingerprint support member having a projection or recess on a surface thereof, it has been heretofore difficult to detect the latent fingerprint with a known composition, whereas with the composition of the present invention, erosion (elimination) or deformation of the latent fingerprint is suppressed, and a general shape thereof is retained, so the latent fingerprint can be efficiently detected. Therefore, the latent fingerprint detecting composition of the present invention is suitable for a wide range of specimens in the field of identification.

## Claims

1. A composition for detecting a latent fingerprint **characterized in that** the composition comprises a 2-cyanoacrylate, a silicone oil and a fluorescent dye, and that a content of the silicone oil is in a range from 700 to 9000 parts by mass based on 100 parts by mass of the 2-cyanoacrylate.

2. The composition for detecting a latent fingerprint according to claim 1, wherein the silicone oil is an alkylsiloxane having 2 to 5 silicon atoms.

3. The composition for detecting a latent fingerprint according to claim 2, wherein the fluorescent dye is at least one selected from a group consisting of an anthraquinone-based dye and a naphthalimide-based dye.

4. The composition for detecting a latent fingerprint according to claim 1, further comprising a stabilizer.

5. The composition for detecting a latent fingerprint according to claim 4, wherein the stabilizer is at least one selected from a group consisting of a sulfurous acid gas, methanesulfonic acid, ethanesulfonic acid, HBF₄, a BF₃ ether complex salt, a BF₃ acetate complex salt, hydroquinone, hydroquinone monomethyl ether, 2,6-di-tert-butyl-p-cresol, trifluoromethanesulfonic acid, and carbon disulfide.

## Patentansprüche

1. Zusammensetzung zum Nachweisen eines latenten Fingerabdrucks, **dadurch gekennzeichnet, dass** die Zusammensetzung ein 2-Cyanoacrylat, ein Silikonöl und einen Fluoreszenzfarbstoff umfasst, und, dass ein Gehalt des Silikonöls in einem Bereich von 700 bis 9000 Gewichtsteilen basierend auf 100 Gewichtsteilen des 2-Cyanoacrylats ist.

2. Zusammensetzung zum Nachweisen eines latenten Fingerabdrucks gemäß Anspruch 1, wobei das Silikonöl ein Alkylsiloxan mit 2 bis 5 Siliciumatomen ist.

3. Zusammensetzung zum Nachweisen eines latenten Fingerabdrucks gemäß Anspruch 2, wobei der Fluoreszenzfarbstoff zumindest einer ist ausgewählt aus einer Gruppe bestehend aus einem Anthrachinon-basierten Farbstoff und einem Naphthalimid-basierten Farbstoff.

4. Zusammensetzung zum Nachweisen eines latenten Fingerabdrucks gemäß Anspruch 1, ferner umfassend einen Stabilisator.

5. Zusammensetzung zum Nachweisen eines latenten Fingerabdrucks gemäß Anspruch 4, wobei der Stabilisator zumindest einer ist ausgewählt aus einer Gruppe bestehend aus einem schweflige Säure-Gas, Methansulfonsäure, Ethansulfonsäure, HBF₄, einem BF₃-Etherkomplex-Salz, einem BF₃-Acetatkomplex-Salz, Hydrochinon, HydrochinonMonomethylether, 2,6-Di-tert-butyl-p-kresol, Trifluormethansulfonsäure, und Kohlenstoffdisulfid.

## Revendications

1. Composition pour détecter une empreinte digitale latente, **caractérisée en ce que** la composition comprend un 2-cyanoacrylate, une huile de silicone et un colorant fluorescent, et **en ce que** la teneur en l'huile de silicone est située dans la plage allant de 700 à 9000 parties en masse pour 100 parties en masse du 2-cyanoacrylate.

2. Composition pour détecter une empreinte digitale latente selon la revendication 1, dans laquelle l'huile de silicone est un alkylsiloxane ayant 2 à 5 atomes de silicium.

3. Composition pour détecter une empreinte digitale latente selon la revendication 2, dans laquelle le colorant fluorescent est au moins l'un choisi dans le groupe constitué par un colorant à base d'anthraquinone et un colorant à base de naphtalimide.

4. Composition pour détecter une empreinte digitale latente selon la revendication 1, comprenant en outre un stabilisant.

5. Composition pour détecter une empreinte digitale latente selon la revendication 4, dans laquelle le stabilisant est au moins l'un choisi dans le groupe constitué par un acide sulfureux gazeux, l'acide méthanesulfonique, l'acide éthanesulfonique, HBF₄, un sel complexe d'éther et de BF₃, un sel complexe d'acétate et de BF₃, l'hydroquinone, l'éther monométhylique d'hydroquinone, le 2,6-di-tert-butyl-p-crésol, l'acide trifluorométhanesulfonique, et le disulfure de carbone.
